# EUROPEAN PATENT APPLICATION

(11) **EP 1 795 537 A1**
(43) Date of publication of application: **13.06.2007**
(21) Application number: 05774616.6
(22) Date of filing: 25.08.2005
(51) Int. Cl.: C07H 21/00, B01J 20/24, B01J 20/28

(54) **LIQUID CRYSTALLINE GEL AND METHOD FOR MANUFACTURING THE SAME**

(30) Priority: 30.08.2004 JP 2004249639
(71) Applicant: NATIONAL UNIVERSITY CORPORATION GUNMA UNIVERSITY, 4-2, Aramaki-machi, Maebashi-shi Gunma 3710044 (JP)
(72) Inventor: DOBASHI, Toshiaki, Ashikaga-shi, Tochigi 3260831 (JP); YAMAMOTO, Takao, Kiryu-shi, Gunma 3760001 (JP); KITA, Erika, nagawa 2400033; (JP); FURUSAWA, Kazuya, Maebashi-shi, Gunma 3710812 (JP); MINAMISAWA, Yoshiyuki, Kiryu-shi, Gunma 3760601 (JP)
(74) Representative: Hart Davis, Jason
(86) International application number: PCT/JP2005/015395
(87) International publication number: WO 2006/025244

(57) **Abstract**

The present invention intends to inexpensively mass-produce a water-insoluble crystalline gel that mainly comprises a nucleic acid having both gelling properties and liquid-crystallizing properties as well as both an adsorptivity of an aromatic compound and optical properties.

A nucleic acid dissolved solution is prepared by dissolving a nucleic acid in water, a buffer, an aqueous solution containing salt, an aqueous polar solvent solution, or a mixture thereof; and the nucleic acid dissolved solution is then dialyzed in an aqueous solution containing a multivalent metal cation having a valence of 2 or more to obtain a liquid crystalline gel mainly comprises a nucleic acid in the shape of a column, tube, cone, rod, fiber, ball, plate, or film. This liquid crystalline gel has a structure, which is concentric and radially oriented from the center when observation is conducted on the plate or film surface.

## Description

### TECHNICAL FIELD

The present invention relates to a gel having a water-insoluble liquid crystalline structure mainly containing nucleic acids typified by deoxyribonucleic acid (DNA) (hereinafter, referred to as "liquid crystalline gel") and a method for manufacturing such a gel by dialysis. More specifically, the present invention relates to a gel having the abilities to adsorb endocrine disruptors, carcinogens, and so on and a method for manufacturing such a gel.

### BACKGROUND ART

It is well known that DNA has a double-helix structure. DNA in aqueous solution is classified as a rod-like polymer having a persistence length of about 500 Å with a minus charge every about 1.5 Å. It is also known that the layer of nucleobase pairs of DNA has a selective adsorptivity of an aromatic compound.
Conventionally, there has been disclosed a method for immobilizing a water-insoluble cross-linked polymer of water-soluble DNA on a support by irradiation of UV light at a wavelength of 250 to 270 nm on an aqueous solution of water-soluble DNA (e.g., DNA derived from salmon testes) or a liquid film thereof on a support, or a thin film obtained by condensation or dryness of a water-soluble DNA thin layer on a support or a liquid film of a solution of water-soluble DNA (e.g., see Patent Document No. 1). According to the method, the irradiation of UV light causes a crosslinking reaction in DNA derived from salmon testis to make the DNA water-insoluble, and this insolubilization technique enables the DNA to adsorb endocrine disruptors composed of aromatic compounds.
Patent Document No. 1: Unexamined Japanese Application No. 2001-810098 A (Claims, Paragraph Nos. 0019 and 0022)

### DISCLOSURE OF INVENTION

### PROBLEM TO BE SOLVED BY THE INVENTION

Unfortunately, however, the method described in Patent Document No. 1 has been inappropriate for a large-scale production because it requires the irradiation of UV light on DNA for a prolonged time to initiate the crosslinking reaction of DNA. Moreover, this method does not produce a liquid crystalline gel like the one of the present invention in which a DNA molecule itself has both gelling properties and liquid-crystallizing properties.

A first object of the present invention is to provide a water-insoluble crystalline gel that mainly comprises a nucleic acid having both gelling properties and liquid-crystallizing properties and to provide a method for manufacturing such a crystalline gel.
A second object of the present invention is to provide a crystalline gel that comprises a nucleic acid having an adsorptivity of an aromatic compound and optical properties and to provide a method for manufacturing such a crystalline gel.
A third object of the present invention is to provide a method for inexpensively manufacturing a liquid crystalline gel mainly comprising a nucleic acid while being producible in large quantities.

### MEANS FOR SOLVING PROBLEM

The invention claimed in claim 1 of the present application is a gel having a crystalline structure in the shape of a column, a tube, a cone, a rod, a fiber, a ball, a plate, or a film, which is made of a nucleic acid, both a nucleic acid and a rod-like polymer, both a nucleic acid and a semi-flexible polymer, or a combination of a nucleic acid, a rod-like polymer and a semi-flexible polymer.
The term "liquid crystalline gel" used herein refers to one in which nucleic acid is agglomerated not only in a liquid crystalline state but also in a gel state.

The invention claimed in claim 13 of the present application is a method for manufacturing a gel, comprising the steps of; preparing a nucleic acid dissolved solution by dissolving a nucleic acid in water, a buffer, an aqueous solution containing salt, an aqueous solution of a polar solvent, or a mixture thereof; and dialyzing the nucleic acid dissolved solution in an aqueous solution containing a multivalent metal cation having a valence of 2 or more, thereby obtaining a gel having a liquid crystalline structure mainly containing the nucleic acid.

### EFFECT OF THE INVENTION

The liquid crystalline gel that mainly contains a nucleic acid as claimed in claim 1 is formed in the shape of a column, tube, cone, rod, fiber, ball, plate, or film and radially oriented from the center thereof when observation is conducted on the cross section perpendicular to longitudinal direction, a radial cross section or the plate surface or the film surface. Therefore, firstly this orientation causes the anisotropical diffusion of the aromatic compound when the liquid crystalline gel is brought into contact with an aromatic compound, and the aromatic compound is then intercalated (inserted) into a gel, thereby forming an intercalation compound on which the aromatic compound is adsorbed. Secondly, the liquid crystalline gel has a liquid crystalline structure and specific optical properties, so that the presence or absence of the adsorption of the aromatic compound can be optically detected. Thirdly, when the liquid crystalline gel is brought into contact with an endocrine disruptor, the endocrine disruptor is intercalated into the gel in a manner similar to the above aromatic compound. Fourthly, when the liquid crystal is brought into contact with a carcinogen, the carcinogen is intercalated into the gel in a manner similar to the aromatic compound, thereby being adsorbed. Fifthly, when the liquid gel is brought into contact with a heavy metal ion, a complex-forming reaction occurs between the heavy metal ion and DNA, thereby adsorbing the heavy metal ion. Sixthly, when a stress is applied on the liquid crystalline gel, the gel elongates and changes its degree of orientation. Furthermore, seventhly, it can be disintegrated under acidic conditions in human body's environment as the liquid crystalline gel contains an ester linkage. Eighthly, it can be disintegrated by microorganisms in the environment as the liquid crystalline gel is biodegradable.

In the method claimed in claim 13 of the present invention, a nucleic acid dissolved solution is prepared by dissolving a nucleic acid in water, a buffer, an aqueous solution containing salt, an aqueous polar solvent solution, or a mixture thereof; and the nucleic acid dissolved solution is then dialyzed in an aqueous solution containing a multivalent metal cation having a valence of 2 or more to obtain a liquid crystalline gel in the shape of a column, tube, cone, rod, fiber, ball, plate, or film. This liquid crystalline gel is insoluble in water and is characterized in that a nucleic acid itself has both gelling properties and liquid- crystallizing properties.
In the production, the amount of the aromatic compound adsorbed in the liquid crystalline gel and the optical properties of the liquid crystalline gel can be controlled by changing the concentration of a buffer or the like, the concentration of a water-soluble nucleic acid, and the concentration of a multivalent metal cation having a valence of 2 or more. Furthermore, a water-insoluble liquid crystalline gel can be obtained by a simple operation in which a nucleic acid dissolved solution is dialyzed in an aqueous solution containing a multivalent metal cation having a valence of 2 or more. This procedure has the feature of being suitable for mass production.

### BRIEF DESCRIPTION OF THE DRAWINGS

Fig. 1 is a schematic cross sectional view showing the molecular structure of a cylindrical liquid crystalline gel in accordance with an embodiment of the present invention.
Fig. 2 is a view obtained by observing the liquid crystalline gel of Fig. 1 under crossed nicols.
Fig. 3 is a view obtained by observing eight liquid crystalline gels of Fig. 1 under crossed nicols.
Fig. 4 is a photograph showing a spherical liquid crystalline gel having a concentric multilayered structure.
Fig. 5 is a photograph showing a fibrous liquid crystalline gel formed in Example 9.

### BEST MODE FOR CARRYING OUT THE INVENTION

Hereinafter, the best mode of the present invention will be described.
The main component of the liquid crystalline gel of the present invention is a nucleic acid. The nucleic acid may be DNA or a mixture of DNA and RNA. For increasing the degree of orientation, the molecular weight of DNA or RNA of the present invention is preferably in the range of 500,000 to 10,000,000. If the molecular weight is less than 500,000, no orientation occurs. If the molecular weight is higher than 10,000,000, an increase in viscosity occurs resulting in difficulty in handling, and the degree of orientation is undesirably kept constant. If the molecular weight is within the above range, the degree of orientation increases as the molecular weight increases. On the other hand, if the molecular weight increases beyond the upper limit, the liquid crystalline gel becomes so hard that it is difficult to process it. Therefore, a liquid crystalline gel having an appropriate processability and a high degree of orientation can be prepared by mixing a nucleic acid having a larger molecular weight with a nucleic acid having a smaller molecular weight. The DNA and RNA of the present invention are water-soluble. The DNA is a single- or double-stranded DNA and is exemplified by DNA derived from testis of a fish, the thymus of a bird or a mammal. In particular, DNA derived from the salmon's testis as one from the fish testis or DNA derived from E. coli is a preferable material for a DNA liquid crystalline gel in terms of quality and cost. Moreover a preferable material for a DNA liquid crystalline gel is any one of DNAs obtained from the thymus glands of a chicken, a caw, and a pig among DNAs from the thymus glands of mammals and birds, or any one of synthetic oligo nucleotides and polynucleotides. The RNA is also exemplified by one derived from yeast.

Furthermore, the liquid crystalline gel may be a combination of a nucleic acid and a rod-like polymer, a combination of a nucleic acid and a semi-flexible polymer, or a combination of a nucleic acid, a rod-like polymer and a semi-flexible polymer. The weight ratio of the components in any of these combinations is 0.1 to 400% by weight of the rod-like polymer and 0.1 to 150% by weight of the semi-flexible polymer in relation to 100% by weight of the nucleic acid. The liquid crystalline gel is in the shape of a column, a tube, a cone, a rod, a fiber, a ball, a plate, or a film. The liquid crystalline gel is configured in a two or three-dimensional structure.
If the liquid crystalline gel is formed in the shape of a column, a tube, a cone, a rod, or a fiber, the liquid crystalline gel mainly contains a nucleic acid radially oriented from the center thereof when observation is conducted on the cross section thereof perpendicular to the longitudinal direction. The liquid gel includes a gel having a concentric multilayered structure when observation is conducted on a cross section perpendicular to a longitudinal direction.
Furthermore, if it is formed in the shape of a ball, the liquid crystalline gel mainly contains a nucleic acid radially oriented from the center thereof when observation is conducted on the radial cross section thereof. Such a liquid crystalline gel include a gel having a concentric multilayered structure when observation is conducted on the radial cross section thereof.

Furthermore, if it is formed in the shape of a plate or a film, the liquid crystalline gel mainly contains a nucleic acid radially oriented from the center thereof when observation is conducted on the plane surface or film surface thereof. Such a liquid crystalline gel include a gel having a concentric multilayered structure when observation is conducted on the plane surface or film surface thereof.
The rod-like polymer or semi-flexible polymer contained in the gel may be a polysaccharide, a polysaccharide derivative, or a combination thereof. Examples of polysaccharide include a fermented polysaccharide (curdlan) and regenerated curdlan (paramylum) produced by microorganisms, and sizofiran produced by Schizophyllum. Examples of polysaccharide derivatives include cellulose derivatives, such as methylcellulose, hydroxypropyl cellulose, hydroxypropyl methylcellulose, and carboxyl methylcellulose.
Another rod-like polymer or semi-flexible polymer in the gel may be a protein, a denatured protein, a polyamino acid, or a combination thereof. Examples of protein include collagen and myosin, examples of denatured protein include gelatin and denatured albumin, and examples of polyamino acid include polyglutamic acid and polylysine.

Furthermore, another rod-like polymer or semi-flexible polymer in the gel may be a synthetic polymer. Examples of synthetic polymer include polyethylene glycol, polyethylene oxide, polyvinyl alcohol, polyvinyl methyl ether, polyvinyl pyrrolidone, polyacrylic acid, and polystyrene sulfonate.
For instance, when DNA is mixed with a synthetic polymer of polyacrylic acid or a polysaccharide of curdlan, liquid crystallinity and carcinogen-adsorbing ability are decreased. In contrast, polyacrylic acid or curdlan, which cannot be decomposed in the living body, adsorbs a carcinogen therein so that they can be utilized as gel beads to be discharged from the living body, e.g., from the anus, or can control biodegradability in the living body and the environment. For forming a liquid crystalline gel from a mixture of DNA and polyacrylic acid (PAA), the weight ratio (DNA/PAA) for mixing DNA with polyacrylic acid in aqueous solution is in the range from 1/4 to 1/0.001. For forming a gel having a highly-oriented liquid crystalline structure, the weight ratio (DNA/PAA) is preferably in the range from 1/1 to 1/0.001. For forming a liquid crystalline gel from a mixture of DNA and polyvinyl alcohol (PVA), the weight ratio (DNA/PVA) for mixing DNA with polyacrylic acid in aqueous solution is in the range from 2/3 to 1/0.001. For forming a gel having a highly-oriented liquid crystalline structure, the weight ratio (DNA/PVA) is preferably in the range from 3/2 to 1/0.001.

Basically, any of liquid crystalline gels mainly containing nucleic acids can be obtained by preparing a nucleic acid dissolved solution by dissolving a nucleic acid in water, a buffer, an aqueous solution containing salt, an aqueous polar solvent solution, or a mixture thereof; and dialyzing the nucleic acid dissolved solution in an aqueous solution containing a multivalent metal cation having a valence of 2 or more.
The nucleic acid dissolved solution may contain either of the above rod-like polymer or the above semi-flexible polymer or may contain both of them in addition to the nucleic acid. Regarding the amount of the rod-like or semi-flexible polymer dissolved in the nucleic acid dissolved solution, in relation to 100% by weight of the nucleic acid, the rod-like polymer dissolved is in the range from 0.1 to 400% by weight and the semi-flexible polymer dissolved is in the range of 0.1 to 150% by weight. By containing the rod-like polymer and the semi-flexible polymer in addition to the nucleic acid, the effects of enzyme stability and acid-base stability can be expected.
A solution for dissolving a nucleic acid is preferably a buffer because the pH thereof can be adjusted. For instance, in case of immersing or dialyzing in an aqueous solution containing a trivalent cation such as aluminum, any of aqueous solutions with a wide range of pH values including water can be used. In case of immersing or dialyzing in an aqueous solution containing a divalent cation such as cobalt salt, a liquid crystalline gel is only generated when a dissolving solution with alkaline pH is used.

Examples of the buffer include: a potassium chloride - hydrochloric acid buffer, a p-toluenesulfonic acid-p-sodium buffer, a glycine - hydrochloric acid buffer, phthalic acid potassium - hydrochloric acid buffer, a citric acid - sodium phosphate buffer, a citric acid - citric sodium buffer, α β,β'-dimethylglutaric acid - sodium hydroxide buffer, an acetic acid - sodium acetate buffer, a succinic acid - sodium hydroxide buffer, a phthalic acid potassium - sodium hydroxide buffer, a sodium cacodylate - hydrochloric acid buffer, a sodium maleate - sodium hydroxide buffer, a phosphate buffer imidazole - hydrochloric acid buffer, a 2:4:6-trimethyl pyridine - hydrochloric acid buffer, a triethanol amine, a hydrochloric acid - sodium hydroxide buffer, a veronal (5:5-sodium diethylbarbiturate) - hydrochloric acid, an N-ethyl morpholine - hydrochloric acid buffer, a Tris buffer, a glycylglycine - sodium hydroxide buffer, a 2-amino-2-methyl 1:3-propanediol-hydrochloric acid buffer, a diethanol amine - hydrochloric acid buffer, a boric acid buffer, a sodium borate - hydrochloric acid buffer, a glycine - sodium hydroxide buffer, a sodium carbonate - a sodium acid carbonate buffer, a sodium borate - sodium hydroxide buffer, a sodium carbonate - sodium hydroxide buffer, a sodium acid carbonate - sodium hydroxide buffer, a sodium dihydrogen phosphate - sodium hydroxide buffer, a potassium chloride - sodium hydroxide buffer, a Britton-Robinson buffer, GTA buffer, and a HEPES buffer.
Examples of the aqueous solution containing salt include an aqueous sodium chloride solution, an aqueous potassium chloride solution, and lithium chloride solution at pH 2 to 10. Furthermore, examples of the aqueous polar solvent solution include an aqueous methanol solution, an aqueous ethanol solution, an aqueous acetone solution, an aqueous formic acid solution, and an aqueous acetic acid solution.

(a) A method for manufacturing a first liquid crystalline gel (method for manufacturing a tubular or columnar liquid crystalline gel)
In the following representative example, a nucleic acid used is a water-soluble DNA and an aqueous solution containing salt used is an aqueous borate solution.
At first, a water-soluble DNA is dissolved in an aqueous borate solution to prepare a nucleic acid dissolved solution. The aqueous borate solution to be used herein is preferably an aqueous solution of sodium tetraborate or sodium borohydride because of retaining the solution at a pH value approximately in the range from pH 9 to 10. The aqueous borate solution exerts a buffering action to retain a pH value of approximately 9, so that the water-soluble DNA can be favorably dissolved therein. For manufacturing the liquid crystalline gel of the present invention, the amount of water-soluble DNA added to the aqueous borate solution is in the range from 0.1 to 2% by weight, preferably from 1 to 1.5% by weight. If the amount of DNA added is less than the lower limit, no liquid crystalline gel is formed. If it exceeds the upper limit, a homogeneous solution is hardly obtained because of an increase in viscosity. Furthermore, since the content of borate salt should be commensurated with the concentration of DNA, the concentration of borate salt is selected in the range of from 0.1 M to a saturated concentration, preferably from 0.02 to 0.04 M. If it is less than 0.01 M, the pH-buffering action is inadequate. For raising the ion strength of the aqueous solution and the solubility of DNA, preferably, alkali metal salt is further added to the aqueous borate solution. The alkali metal salt exemplified by sodium chloride, lithium chloride, or potassium chloride. A sufficient concentration of the alkali metal salt is 0.01 M.

Subsequently, the nucleic acid dissolved solution thus obtained is filled in a dialysis-membrane tube. Prior to filling, the bottom end of the tube is sealed. Examples of the dialysis membrane include, but not specifically limited to, cellulose acetate and polymethyl methacrylate. Among them, a cellulose-based dialysis membrane is preferable. Here, the rate of gelation, and the liquid crystallinity and layer structure of a gel to be obtained vary depending on the film thickness and diameter of the dialytic tube. An outer diameter of the cylindrical liquid crystalline gel finally obtained depends on the diameter of the tube. The diameter and length of the tube can be determined depending on the application of the liquid crystalline gel. For instance, there is obtained a cylindrical liquid crystalline gel where the diameter of a tube is selected in the range from 6 mm to 10 cm in outer diameter and from 0 mm to 4 cm in inner diameter (1 mm to 10 cm in thickness). After filling a nucleic acid dissolved solution, the upper end of the tube is sealed, resulting in a hermetically-sealed nucleic acid dissolved solution in tube. It is to be noted that a liquid crystalline gel as large as possible can be prepared principally by using a dialysis membrane stuck on an appropriate frame instead of a dialytic tube.

Next, the filled and sealed nucleic acid dissolved solution in tube is immersed in an aqueous solution containing a metal cation having a valence of 2 or more, preferably a divalent or trivalent metal cation. A coagulating solution containing a multivalent metal cation having a valence of 2 or more is used because a nucleic acid dissolved solution gelates in the presence of such a metal cation and causes crystallization in this process. A suitable divalent metal cation to be used herein is any of divalent transition metal cations, such as cobalt, nickel, iron, and manganese in term of mutual action with DNA. A suitable trivalent metal cation is a trivalent aluminum cation and a lanthanide dysprosium cation in terms of obtaining a liquid crystalline gel having a higher transparency. Such a compound is exemplified by a salt that allows the above metal ion to dissociate therefrom in an aqueous solution and examples thereof include cobalt chloride, cobalt sulfate, nickel chloride, nickel sulfate, iron chloride, ferrous sulfate, manganese chloride, manganic sulphate, aluminum chloride, aluminum sulfate, and dysprosium chloride.

The concentration of the aqueous solution containing a metal cation is preferably in the range of from 0.1 M to a saturated concentration, more preferably from 0.1 to 0.2 M. If it is less than the lower limit, no gel can be formed. If it exceeds the upper limit, undesired results such as a distorted liquid crystal occur due to significant shrinkage of a gel.
Furthermore, the temperature of the aqueous solution is preferably in the range of 0 to 40°C, more preferably from 30 to 40 °C. If the temperature is less than the lower limit, the diffusion rate of the solution is lowered resulting in undesired solidification. If it exceeds the upper limit, no formation of a liquid crystalline gel may occur or undesired results such as the break down of a liquid crystalline gel structure once formed may occur. When the dialysis-membrane tube is immersed in an aqueous solution containing a multivalent metal cation having a valence of 2 or more, boronic acid of DNA in the dialysis-membrane tube disperses outside of the tube, while the dissociated multivalent metal cation having a valence of 2 or more on the outside disperses into the tube.

The above dialysis forms a cylindrical body in the tube. The cylindrical body formed has a diameter corresponding to the diameter of the tube. In general, the center of the cylindrical body is solated and the periphery thereof is formed in liquid crystalline gel by shortening the duration of dialysis, lowering the concentration of nucleic acid, or lowering the concentration of the aqueous solution containing a multivalent metal cation having a valence of 2 or more. In contrast, a solid cylindrical liquid crystalline gel can be formed by extending the duration of dialysis, increasing the concentration of nucleic acid, or increasing the concentration of the aqueous solution containing a multivalent metal cation having a valence of 2 or more. That is, when the center of the cylindrical body is sol, it is taken out from the tube and then rinsed with water to remove a sol part out, thereby obtaining a tubular liquid crystalline gel. In addition, when the whole part of a cylindrical body is gelled, the cylindrical body is taken out from the tube and then rinsed with water, thereby obtaining a solid columnar liquid crystalline gel. For example, under the conditions of 1 part by weight of DNA, 0.02 M of boronic acid and 0.1 M of cobalt chloride, a tubular liquid crystalline gel is formed at a dialysis time in the range from 0 to 30 minutes and a columnar liquid crystalline gel is formed at a dialysis time in the range from 60 minutes or more.

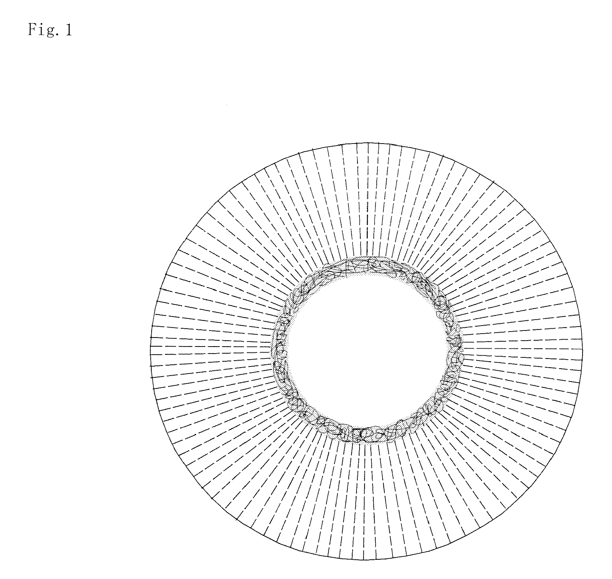

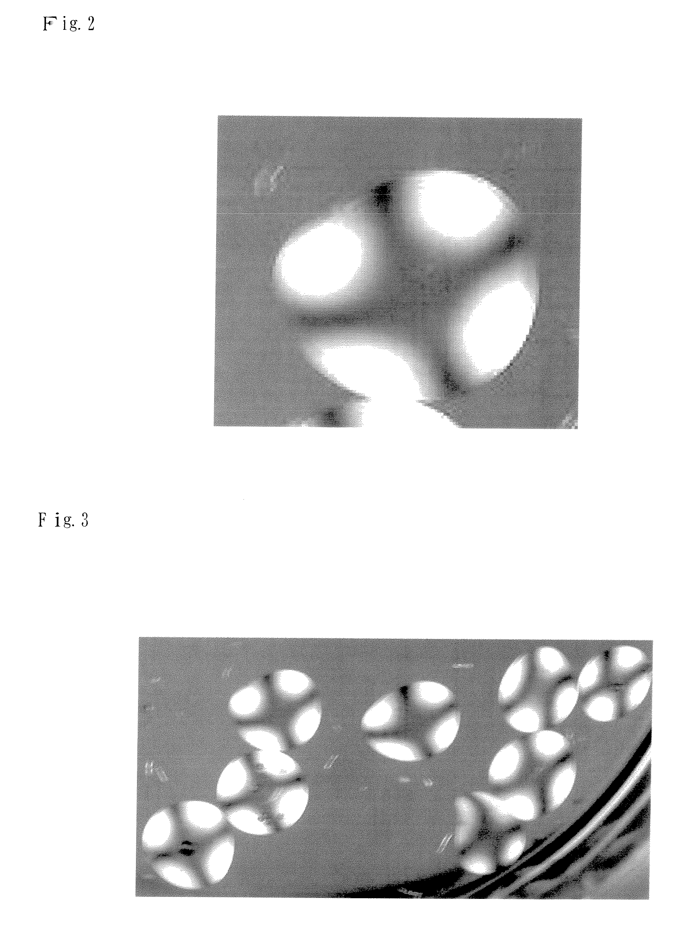

When a tubular liquid crystalline gel is cut perpendicularly to the longitudinal direction and the cut surface is observed under natural light, it is concentrical and radially oriented from the center like the transverse section of a pine apple fruit as shown in the schematic diagram of Fig. 1. In contrast, when it is observed under crossed nicols, as shown in Figs. 2 and 3, a cross line appears, demonstrating the presence of a liquid crystalline structure in the gel. The cross line demonstrates the DNA molecule or the agglomerate of DNA molecules is regularly oriented from the center thereof. Even though the mechanism of obtaining such a structure has not been revealed at the present stage, it is considered the formation of such structure is caused by a change in DNA structure by replacement of a solvent and the subsequent formation of a metal cation/DNA complex.
In addition, the following procedure is carried out to make the tubular or columnar liquid crystalline gel into one showing a concentric multilayered structure when observation is conducted on the cross section thereof perpendicular to the longitudinal direction. At first, a cylindrical body is formed in a tube by placing the tube in an aqueous borate solution or in an aqueous solution containing an alkali metal salt in a borate salt. Next, the tube is taken out from the aqueous solution into the atmosphere and left to stand for 5 to 30 minutes. Subsequently, the tube is then immersed in the above aqueous solution to dialyze the nucleic acid dissolved solution. Repetition of taking out the tube from the aqueous solution and immersing the tube in the aqueous solution prevents the structural formation by the inflow of cation to form a tubular or columnar gel having a concentric multilayered structure in the tube when observation is conducted on the cross section perpendicular to the longitudinal direction.

(b) Method for manufacturing a second liquid crystalline gel (method for manufacturing a spherical liquid crystalline gel)
In the following representative example, a nucleic acid used is a water-soluble DNA and an aqueous solution containing salt used is an aqueous borate solution in a manner similar to the first manufacturing method.
At first, nucleic acid dissolved solution and an aqueous solution containing a metal cation having a valence of 2 or more, preferably a divalent or trivalent metal cation are prepared, respectively. The concentration of an aqueous borate solution and the content of DNA for preparing the nucleic acid dissolved solution are different from those employed in the first manufacturing method in that there is a need of providing the nucleic acid dissolved solution with a viscosity enough to retain its spherical shape by boundary tension during the formation of such a dialysis membrane for allowing the self formation of a dialysis membrane. That is the concentration of an aqueous borate solution is in the range from 0.05 M to a saturated concentration, preferably from 0.01 to 0.08 M. The concentration of an aqueous salt solution containing a multivalent metal cation having a valence of 2 or more should be 0.02 M or more because a cross-linkage should be immediately formed to allow the self formation of a dialysis membrane. That is the concentration of a multivalent metal cation having a valence of 2 or more in the aqueous solution is in the range from 0.02 M to a saturated concentration, preferably from 0.1 to 2 M. The temperature of the aqueous borate solution and the temperature of aqueous solution containing salt of metal cation are the same as those employed in the first manufacturing method.
In the second manufacturing method, a syringe, a nozzle, a spray, or a micropipette is used in substitution for the dialysis-membrane tube. An outer diameter of the spherical liquid crystalline gel to be finally obtained depends on the aperture of a discharge orifice of a syringe or the like. The aperture of the discharge orifice of the syringe or the like is defined on the basis of the application of a liquid crystalline gel. For instance, the aperture of the discharge orifice can be selected in the range from 1 µm to 1 mm. Such a syringe or the like allows a spherical liquid gel having an outer diameter of 100 *µ*m to 4 mm to be obtained. After filling the nucleic acid dissolved solution into the syringe or the like, the discharge orifice is turned downward and the syringe or the like is then fixed at a predetermined position 1 to 15 cm above the level of the aqueous solution containing a multivalent metal cation having a valence of 2 or more.

Subsequently, the nucleic acid dissolved solution filled in the syringe or the like is dropped into the aqueous solution containing a multivalent metal cation having a valence of 2 or more by applying pressure on the inside of the syringe or the like. While the nucleic acid dissolved solution in the shape of a liquid droplet migrates in the aqueous solution, a dialysis membrane is formed on the entire circumference of a liquid droplet.
The dialysis membrane consists of a DNA gel induced by the metal cation. By retaining such a state, the nucleic acid dissolved solution in the shape of the liquid droplet is dialyzed and a spherical liquid crystalline gel is then formed in the inside of the dialysis membrane. Consequently, there is formed a spherical liquid crystalline gel having an outer diameter corresponding to the aperture of the discharge orifice of the syringe or the like.
When the spherical liquid crystalline gel is cut in the diameter direction to obtain hemispheres and the cross section thereof is then observed, the spherical liquid crystalline gel has an outer shell and is concentric and radially oriented from the center (not illustrated). In the center of the inside of the liquid crystalline gel, there is an ungelled sol portion. A mechanism for forming such a structure is the same as the tubular liquid crystalline gel.

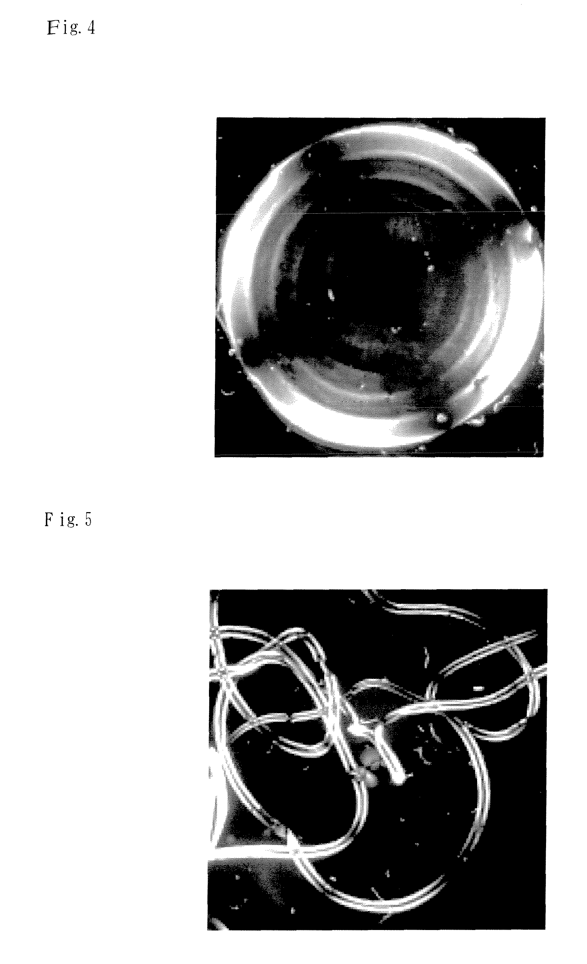

Furthermore, for obtaining a concentric multilayered structure when observation is conducted on the radial cross section of a spherical liquid crystalline gel, the following procedure is carried out. At first, a dialysis membrane is formed on the entire circumference of a liquid droplet in an aqueous borate solution or in an aqueous solution containing an alkaline metal salt in borate salt. Next, a spherical gel forming a dialysis membrane on the entire circumference thereof is taken out from the aqueous solution into the atmosphere and then left to stand for 5 to 30 minutes. Subsequently, the spherical gel is immersed in the above aqueous solution and the nucleic acid dissolved solution is then dialyzed. Repetition of taking out the spherical gel from the aqueous solution and immersing the spherical gel in the aqueous solution prevents the structural formation by the inflow of cation. As a result, a spherical liquid crystalline gel having a concentric multilayered structure observed when observation is conducted on the radial cross section, can be formed in a dialysis membrane. Fig. 4 shows a spherical liquid crystalline gel having a concentric multilayered structure. When a drug is placed in a gel or in a sol layer in the inside thereof and the gel is then taken in the living body, the drug can be gradually released in the living body. In other words, when the drug in the gel or in the outer layer of the sol layer of the gel is released in the body, the drug surrounded by the inner layer next to the outer layer migrates to the outer layer, and then released from the outer layer into the body. As the drug migrates from the inner layer to the outer layer, the drug entered in the body can be gradually released.

(c) Method for manufacturing a third liquid crystalline gel (method for manufacturing rod-like or fibrous liquid crystalline gel)
At first, water-soluble DNA is dissolved in water to prepare a nucleic acid dissolved solution. The nucleic acid dissolved solution is extruded from a syringe or nozzle into an aqueous solution containing a metal cation having a valence of 2 or more, preferably a divalent or trivalent metal cation to form a dialysis membrane on the entire circumference of a rod-like or fibrous body in the aqueous solution, during dialyzing the nucleic acid dissolved solution. As a result, a gel having a rod-like or fibrous liquid crystalline structure, which is concentric and radially oriented from the center when observation is conducted on the cross section thereof perpendicular to the longitudinal direction, is formed in the dialysis membrane. The gel is taken out from the aqueous solution into the atmosphere and then rinsed with water to obtain a solid or hollow rod-like or fibrous gel. The length and thickness of the fiber can be adjusted by controlling both the extrusion rate and the viscosity of the nucleic acid dissolved solution.
Moreover, for obtaining a concentric multilayered structure when observation is conducted on the cross section of the rod-like or fibrous liquid crystalline gel perpendicular to the longitudinal direction, the following procedure is carried out. At first, a dialysis membrane is formed on the entire circumference of a rod-like or fibrous body in an aqueous borate solution or in an aqueous solution containing an alkaline metal salt in a borate salt. Next, the rod-like or fibrous gel with the dialysis membrane already formed on the entire circumference is taken out from the aqueous solution and then left to stand for 5 to 30 minutes. Subsequently, the rod-like or fibrous gel is immersed in the above aqueous solution to dialyze the nucleic acid dissolved solution. Repetition of taking out the rod-like or fibrous gel from the aqueous solution and immersing the rod-like or fibrous gel in the aqueous solution prevents the structural formation by the inflow of cation. As a result, a rod-like or fibrous liquid crystalline gel having a concentric multilayered structure observed when observation is conducted on the cross section perpendicular to the longitudinal direction can be formed in the inside of a dialysis membrane.

(d) Method for manufacturing a fourth liquid crystalline gel (method for manufacturing plate-shaped or film-shaped liquid crystalline gel)
In the following representative example, a nucleic acid used is a water-soluble DNA and an aqueous solution containing salt used is an aqueous borate solution in a manner similar to the first manufacturing method.
At first, the same nucleic acid dissolved solution as that of the above second manufacturing method and an aqueous solution containing a metal cation having a valence of 2 or more, preferably a divalent or trivalent metal cation are prepared. The nucleic acid dissolved solution is dropped onto a first plate, and then another second plate equal to or smaller than the first one is placed over the first plate to flatten the nucleic acid dissolved solution dropped thereon. The first plate is not particularly limited as far as it is made of a material having a smooth surface and allowing a film formed thereon to be easily peeled off. Examples of the first plate include a glass substrate, a plastic substrate and a ceramic substrate. The second plate is not particularly limited as far as it is a smooth plate for forming the uniform thickness of a nucleic acid dissolved solution (liquid film) and made of a material so as to be easily peeled off of a film. The second plate is preferably a disk-shaped cover glass, an acryl plate, or a polyethylene terephthalate (PET) film. The size of the second plate can be selected in the range from 0.12 to 17 mm in thickness and 15 to 22 mm in diameter. In addition, when an acryl plate or the like is used as the plate instead of the commercial produced cover glass, the size of the plate may be arbitrarily determined.

The thickness of a flattened nucleic acid dissolved solution , i.e., the thickness of a liquid film, is preferably in the range from 0.5 to 2 mm. If it is lower than the lower limit, it is difficult to make a gel. If it exceeds the upper limit, it is difficult to make a gel even though the viscosity of the nucleic acid dissolved solution is increased. Secondly, the flattened nucleic acid dissolved solution in a state of being sandwiched between the first and second plates is immersed in an aqueous solution containing a multivalent metal cation having a valence of 2 or more. Even though it is immersed in the aqueous solution, the second plate is not peeled off of the liquid film because of interaction with the nucleic acid dissolved solution. In this aqueous solution, a dialysis film is formed on a portion (the lateral circumference on liquid film) without being covered with the first and second plates of the flattened nucleic acid dissolved solution (liquid film). The dialysis membrane is made of a DNA gel induced by the metal cation. By retaining such a state, the nucleic acid dissolved solution constituting the liquid film is dialyzed, resulting in the formation of a plate-shaped or film-shaped liquid crystalline gel in the inside of the dialysis membrane. The thickness of the plate-shaped or film-shaped liquid crystalline gel is proportional to that of the liquid film and is in the range from 0.5 to 2 mm. The size thereof is proportional to one of the second plate and is in the shape of a disk having a diameter in the range from 15 to 22 mm. When the size of the plate is arbitrarily defined, a liquid crystalline gel having any size can be produced.

When observation is conducted on the surface of a plate-shaped or film-shaped liquid crystalline gel, but not shown in the figure, the plate-shaped or film-shaped liquid crystalline gel is concentric and radially oriented from the center (not illustrated). In the center of the inside of the liquid crystalline gel, there is an ungelled sol portion. The mechanism of forming such a structure is the same as that of forming the tubular liquid crystalline gel. A second plate is peeled off of the plate-shaped or film-shaped liquid crystalline gel and then the latter is rinsed with water to obtain a plate-shaped or film-shaped liquid crystalline gel from which the center portion is removed.
Furthermore, for obtaining a concentric multilayered structure when observation is conducted on the plate surface or the film surface, the following procedure is carried out. At first, a dialysis membrane is formed on a portion (the lateral circumference on liquid film) without being covered with the first and second plates in an aqueous borate solution or in an aqueous solution containing an alkaline metal salt in borate salt. Secondly, the first and second plates are taken out from the aqueous solution into the atmosphere and then left to stand for 5 to 30 minutes. Subsequently, the first and second plates are immersed in the above aqueous solution to dialyze the nucleic acid dissolved solution. Repetition of taking out the first and second plates from the aqueous solution and immersing the first and second plates in the aqueous solution prevents the structural formation by the inflow of cation. As a result, a plate-shaped or film-shaped liquid crystalline gel having a concentric multilayered structure when observation is conducted on the plate-shaped or film-shaped surface can be formed in the inside of a dialysis membrane.

### Examples

Hereinafter, examples of the present invention will be described together with comparative examples thereof.

### <Example 1>

A nucleic acid dissolved solution containing DNA was prepared by dissolving 1% by weight of DNA powder derived from salmon's testes, which was one of water-soluble DNAs, in a buffer at 50°C where 40 mM of sodium tetraborate and 20 mM of sodium chloride salt were dissolved in water. The nucleic acid dissolved solution was injected into each of four different cellulose dialysis tubes having diameters of 6, 16, 20, and 25 mm and the tubes were then sealed, respectively, followed by immersing in a 100 mM aqueous cobalt chloride solution at room temperature to carry out dialysis. After 24-hour dialysis, four cylindrical gels having different diameters with high gel strengths were formed in each of the tubes. As compared with the diameters of the respective dialysis tubes, all of the cylindrical gels had been shrunken in diameter almost by 14 to 15%. When these gels were taken out from the respective tubes and observed, the center portions thereof were sol. When the cylindrical gels were rinsed with water, their respective sol portions were easily removed, thereby obtaining tubular liquid crystalline gels made of DNA, with four different dimensions of 5.1 mm in external diameter and 2.1 mm in inner diameter; 13.0 mm in external diameter and 6.4 mm in inner diameter; 16.6 mm in external diameter and 7.4 mm in internal diameter; and 20.7 mm in external diameter and 9.5 mm in internal diameter. When these gels were cut in round slices in the direction perpendicular to the longitudinal direction and then observed through a polarization lens under crossed nicols, the presence of a liquid crystalline structure in which molecules were oriented in the longitudinal direction was confirmed. It was found that a gel tube made of such a DNA liquid crystalline gel could be prepared without depending on the size of a dialysis tube.

### <Comparative Example 1>

The formation of a cylindrical gel was tried under the same conditions as those of Example 1 except changing the concentration of DNA to 0.1% by weight. However, the sol in the tube remained unchanged so that any liquid crystalline gel made of DNA could not be formed.

### <Example 2>

A nucleic acid dissolved solution containing DNA was prepared by dissolving 1.5% by weight of DNA powder derived from salmon's testes, which was one of water-soluble DNAs, in a buffer at 50°C where 40 mM of sodium tetraborate and 20 mM of sodium chloride salt were dissolved in water. The nucleic acid dissolved solution was filled in a micropipette with an yellow tip (0.5 mm in aperture of a discharge orifice) and then dropped into an aqueous aluminum chloride solution at 25°C in any one of 10 different concentrations (1 mM to 1 M) listed in Table 1, from the position 5 cm above the level of the solution and then the droplet was immersed therein for 5 minutes. In all of the aqueous solutions, except one having an aluminum ion concentration of 0.001 M, the circumference of the above nucleic acid dissolved solution was immediately gelled. The gelled portion acted as a dialysis membrane and a spherical gel having a diameter in the range from about 5 to 8 mm is formed. The presence or absence of liquid crystalline formation with respect to a gel obtained in Example 2 was examined. The results are shown in Table 1.

### <Comparative Example 2>

The formation of a spherical gel was tried under the same conditions as those of Example 2 except changing the concentration of DNA to 0.08% by weight. However, the sol remained unchanged and no gelation occurred regardless of the concentration of aluminum chloride ion.

**[Table 1]**

| Immersion conditions | | Example 2 | | Comparative Example 2 |
|---|---|---|---|---|
| Al conc. [M] | Duration of immersion [sec.] | 15 wt% in DNA conc. | Diameter of gel [mm] | 0.08 wt% in DNA conc. |
| 0.001 | 5 | No liquid crystallization occurred in gel | - | No liquid crystallization occurred in gel |
| 0.01 | 5 | Liquid crystallization occurred in gel | 5 to 8 | No liquid crystallization occurred in gel |
| 0.025 | 5 | Liquid crystallization occurred in gel | 5 to 8 | No liquid crystallization occurred in gel |
| 0.05 | 5 | Liquid crystallization occurred in gel | 5 to 8 | No liquid crystallization occurred in gel |
| 0.1 | 5 | Liquid crystallization occurred in gel | 5 to 8 | No liquid crystallization occurred in gel |
| 0.15 | 5 | Liquid crystallization occurred in gel | 5 to 8 | No liquid crystallization occurred in gel |
| 0.2 | 5 | Liquid crystallization occurred in gel | 5 to 8 | No liquid crystallization occurred in gel |
| 0.4 | 5 | Liquid crystallization occurred in gel | 5 to 8 | No liquid crystallization occurred in gel |
| 0.8 | 5 | Liquid crystallization occurred in gel | 5 to 8 | No liquid crystallization occurred in gel |
| 1.0 | 5 | Liquid crystallization occurred in gel | 5 to 8 | No liquid crystallization occurred in gel |

As is evident from Table 1, in comparative Example 2, no gelation nor crystallization occurred. In Example 2, all samples were gelled except one having an aluminum concentration of 0.001 M. When, among the gelled products, spherical gels prepared by dropping into aqueous aluminum chloride solutions of 10 mM or more in concentrations were observed using a deflecting plate under crossed nicols conditions, it was confirmed that each of them had a liquid crystalline structure. Moreover, as a result of relatively comparing volume shrinkage rates of the respective gelled products, it was found that the higher the concentration of aluminum ion increased, the higher the volume shrinkage rate is.

### <Example 3>

Five different buffers at 50°C were prepared by dissolving sodium tetraborate and sodium chloride in water, where the concentrations of sodium tetraborate salt were 10 mM, 20 mM, 40 mM, 60 mM, and 80 mM and the concentration of sodium chloride was 20 mM. In each of these buffers, 1.0% by weight of DNA powder derived from salmon's testes, which was one of water-soluble DNAs, was dissolved, thereby five different nucleic acid dissolved solutions containing DNA were prepared. Thirty glass substrates were provided and were then arranged in groups of six glass-substrates. Each of the nucleic acid dissolved solutions was dropped on the upper surfaces of the each six glass substrates in one of the six groups and the droplet was then covered with a disk-shaped cover glass of 0.12 to 0.17 mm in thickness and 15 to 22 mm in diameter. As a result, a liquid film having a thickness of about 1 mm was formed between the glass substrate and the cover glass.
On the other hand, six different aqueous cobalt chloride solutions of 50 mM, 100 mM, 200 mM, 400 mM, 600 mM, and 800 mM in cobalt chloride salt concentration were provided as solidification solutions. Six glass substrates in each group were respectively immersed in the six different aqueous cobalt chloride solutions.
Thus, 30 different types of immersion were carried out. In each of the lateral sides of all liquid films, a film was formed by a reaction of the aqueous DNA solution (nucleic acid dissolved solution) with cobalt ions. The film acted as a dialysis membrane and the subsequent dispersion of cobalt ions through the dialysis membrane allowed the formation of a water-insoluble film between the glass substrate and the cover glass. When the cover glass was removed, disk-shaped films in the range from 0.12 to 0.17 mm in thickness and 15 to 22 mm in diameter were obtained on the glass substrate. When all of 30 different films were observed using a deflecting plate under crossed nicols conditions, it was confirmed that all films had liquid crystalline structures.

### <Comparative Example 3>

Dialysis was carried out in a manner similar to Example 3, except that the size of a liquid droplet was reduced and the thickness of a liquid film was about 1 *µ*m. However, no gelation occurred between a glass substrate and a cover glass and sol remained unchanged.

### <Comparative Example 4>

A nucleic acid dissolved solution containing DNA was prepared by dissolving 0.1% by weight of DNA powder derived from salmon's testes, which was one of water-soluble DNAs, in relation to 100% by weight of a buffer (50°C) where 40 mM of sodium tetraborate and 20 mM of sodium chloride in water. The nucleic acid dissolved solution was dropped on a glass substrate in a similar manner to that of Example 3 and the liquid droplet was then covered with the same of a cover glass as that of Example 3. In this state, it was immersed in an aqueous cobalt chloride solution (100 mM) to carry out dialysis at room temperature. However, no gelation occurred between the glass substrate and the cover glass and sol remained unchanged.

### <Example 4>

The spherical liquid crystalline gel obtained in Example 2 was immersed in one of carcinogens, an aqueous acridine orange solution. As a result, it was confirmed that the spherical liquid crystalline gel made of DNA had an ability of adsorbing an acridine orange molecule. The adsorption of acridine orange to DNA may be considered due to intercalation and thus it is expected endocrine disruptors and typical carcinogens, which are characterized by having planar aromatic rings, can be also adsorbed. Consequently, the DNA liquid crystalline beads can be used as agents for adsorbing endocrine disruptors. Furthermore, it is also possible to use a hollow cylindrical liquid crystalline gel in the same kind of application.

### <Example 5>

The film-shaped liquid crystalline gel obtained in Example 3 was employed as a quantitative indicator for aromatic compounds, such as endocrine disruptors. In other words, the degree of coloration or the degree of light transmission of film-shaped liquid crystalline gel before making contact with a sample containing an aromatic compound was compared with the degree of coloration or the degree of light transmission of film-shaped liquid crystalline gel after making contact with a sample containing an aromatic compound, thereby the quantitative measurement of an aromatic compound, such as an endocrine disruptor, was allowed by the degree of coloration or the degree of light transmission.

### <Example 6>

A liquid crystalline gel was formed by mixing a water-soluble DNA with curdlan. At first, 1% by weight of DNA powder derived from salmon's testes, which was one of water-soluble DNAs, and 5% by weight of curdlan were dissolved in a buffer at 50°C prepared by dissolving 20 mM of sodium tetraborate and 400 mM of sodium hydroxide in water, thereby a nucleic acid dissolved solution containing DNA and curdlan was prepared. Subsequently, the nucleic acid dissolved solution was dropped in a 100-mM aqueous cobalt chloride and aluminum chloride solution using a micropipette. The nucleic acid - curdlan mixture dissolved solution dropped in both the aqueous solutions of metal cations was immediately gelled to form gel beads. After five minutes passed, these gel beads were observed using a polarization plate under crossed nicols conditions. As a result, in each of both aqueous metal cation solutions, gel beads having a liquid crystalline structure were formed.

### <Example 7>

Liquid crystalline gel beads made of DNA and curdlan were obtained in a similar manner to that of Example 6, except that a nucleic acid dissolved solution was prepared by dissolving 1% by weight of DNA powder derived from salmon's testes and 3% by weight of curdlan.

### <Example 8>

Liquid crystalline gel beads made of DNA and curdlan were obtained in a similar manner to that of Example 6, except that a nucleic acid dissolved solution was prepared by dissolving 0.5% by weight of DNA powder derived from salmon's testes and 5% by weight of curdlan.
The liquid crystalline gel beads obtained in Examples 6 to 8 were characterized in that they were hardly dissolved in an aqueous DNA degradative enzyme (DNase) solution.

### <Example 9>

A nucleic acid dissolved solution was prepared by dissolving a water-soluble DNA in water at any of concentrations (0.2 to 1% by weight) listed in Table 2. The nucleic acid dissolved solution was extruded from a nozzle with 0.5 mm in aperture of a discharge orifice into an aqueous aluminum chloride solution at 25°C at each of two different concentrations (50 mM and 1 M) listed in Table 2 to form a dialysis membrane on the entire circumference of a fibrous body in the aqueous solution, while dialyzing the nucleic acid dissolved solution. Consequently, an anisotropic fibrous liquid crystalline gel could be easily formed. When the fibrous liquid crystalline gels obtained in Example 9 are provided in bundle to form a column, the column can have a manageable configuration and a manageable strength. In Table 2, a gel formed under the conditions of 1.0% by weight of DNA and an aqueous aluminum chloride solution of 100 mM in concentration was a highly-oriented fibrous liquid crystalline gel having a uniform thickness. This fibrous liquid crystalline gel is shown in Fig. 5.

**[Table 2]**

| DNA concentration | Concentration of aqueous AlCl₃ solution | Presence or absence of gelation | Form | Presence or absence of liquid crystallization |
|---|---|---|---|---|
| 0.2 wt% | 50 mM | Presence | Fibrous | Presence |
| 0.2 wt% | 100 mM | Presence | Fibrous | Presence |
| 0.5 wt% | 50 mM | Presence | Fibrous | Presence |
| 0.5 wt% | 100 mM | Presence | Fibrous | Presence |
| 0.7 wt% | 50 mM | Presence | Fibrous | Presence |
| 0.7 wt% | 100 mM | Presence | Fibrous | Presence |
| 1.0 wt% | 50 mM | Presence | Fibrous | Presence |
| 1.0 wt% | 100 mM | Presence | Fibrous | Presence |

### <Example 10>

A liquid crystalline gel was formed by mixing a water-soluble DNA with polyacrylic acid. At first, a nucleic acid dissolved solution containing DNA and polyacrylic acid was prepared by dissolving 1% by weight of DNA powder derived from salmon's testes, which was one of water-soluble DNAs, and 1% by weight of polyacrylic acid (MW = 250,000) in a buffer at 50°C where 20 mM of sodium tetraborate and 400 mM of sodium hydroxide were dissolved in water. Subsequently, the nucleic acid dissolved solution was dropped in a 100 mM aqueous cobalt chloride and aluminum chloride solution using a micropipette. The nucleic acid - polyacrylic acid mixture dissolved solution dropped in both the aqueous solutions of metal cations was immediately gelled to form gel beads. After five minutes passed, these gel beads were observed using a polarization plate under crossed nicols conditions. Consequently, it was revealed that gel beads having liquid crystalline structures were formed in each of both aqueous metal cation solutions. The liquid crystalline gel beads obtained in Example 10 were characterized in that they were hardly dissolved in acid or base. This fact indicates that it can be applied as gel beads capable of adsorbing carcinogens leased from the living body without being disintegrated.

### <Example 11>

A liquid crystalline gel was formed by immersing a water-soluble DNA in an aqueous polar solvent solution containing a metal cation. At first, a nucleic acid dissolved solution containing DNA was prepared by dissolving 0.7% by weight of DNA powder derived from salmon's testes, which was one of water-soluble DNAs, in a buffer at 25°C where 20 mM of sodium tetraborate and 400 mM of sodium hydroxide were dissolved in water. Subsequently, the nucleic acid dissolved solution was dropped into each of an aqueous ethanol solution, an aqueous methanol solution, and an aqueous acetone solution, containing 100 mM of aluminum chloride using a micropipette. The concentrations of polar solvents were 10% by volume, respectively. The nucleic acid dissolved solution dropped in the aqueous aluminum cation solution was immediately gelled to form gel beads. After five minutes passed, these gel beads were observed using a polarization plate under crossed nicols conditions.
As a result, it was revealed gel beads having liquid crystalline structure were formed.

### Industrial Applicability

Firstly, as the liquid crystalline gel of the present invention has both an adsorptivity of an aromatic compound and optical properties, it is available for an absorbent for an endocrine disruptor. A hollow cylindrical liquid crystalline gel or a spherical liquid crystalline gel is filled in a column and a harmful solution containing an endocrine disruptor is then allowed to pass through the column so that it becomes possible to industrially adsorb endocrine disruptors. Secondly, as most of endocrine disruptors have chromogenic properties, the film-shaped liquid crystalline gel or the above column absorbing an endocrine disruptor shows a change in degree of coloration or degree of light transmission thereof so that it can be used for an indicator of endocrine disruptor. When the endocrine disruptor does not have chromogenic properties, a liquid crystalline gel with crossed nicols arranged on the both sides thereof shows a change in light transmission between before and after adsorption so that it can be also used as an indicator. Thirdly, as most of color pigments expected to be used as organic ELs are low-molecular weight compounds having aromatic ring structures, a device having both the selective light transmission and the functions of crystals can be manufactured by intercalating these color pigments. Fourthly, as it has a metal iron doped, the expandability of the liquid crystalline gel of the present invention as a new conductive material can be desired.

## Claims

1. A gel comprising a nucleic acid, both a nucleic acid and a rod-like polymer, both a nucleic acid and a semi-flexible polymer, or a combination of a nucleic acid, rod-like polymer and a semi-flexible polymer, and having a liquid crystalline structure in the shape of a column, a tube, a cone, a rod, a fiber, a ball, a plate, or a film.

2. The gel according to claim 1, wherein the nucleic acid is DNA or a combination of DNA and RNA.

3. The gel according to claim 1, wherein the gel is formed in the shape of a column, a tube, a cone, a rod, or a fiber and a main component thereof is a nucleic acid radially oriented from the center when observation is conducted on a cross section thereof perpendicular to a longitudinal direction.

4. The gel according to claim 3, wherein the gel has a concentric multilayered structure when observation is conducted on a cross section thereof perpendicular to a longitudinal direction.

5. The gel according to claim 1, wherein the gel is spherically formed and a main component is a nucleic acid radially oriented from the center thereof when observation is conducted on a radial cross section.

6. The gel according to claim 5, wherein the gel has a concentric multilayered structure when observation is conducted on a radial cross section.

7. The gel according to claim 1, wherein the gel is formed in the shape of a plate or a film and a main component is a nucleic acid radially oriented from the center when observation is conducted on a plate or film surface.

8. The gel according to claim 7, wherein the gel has a concentric multilayered structure when observation is conducted on a plate or film surface.

9. The gel according to claim 1, wherein the rod-like polymer or the semi-flexible polymer is a polysaccharide, a polysaccharide derivative, or a combination thereof.

10. The gel according to claim 1, wherein the rod-like polymer or the semi-flexible polymer is a protein, a denatured protein, a polyamino acid, or a combination thereof.

11. The gel according to claim 1, wherein the rod-like polymer or the semi-flexible polymer is a synthetic polymer.

12. The gel according to claim 11, wherein the synthetic polymer is a polyethylene glycol, a polyethylene oxide, a polyvinyl alcohol, a polyvinyl methyl ether, a polyvinyl pyrrolidone, a polyacrylic acid, or a polystyrene sulfonate.

13. A method for manufacturing a gel, comprising the steps of:
preparing a nucleic acid dissolved solution by dissolving a water-soluble nucleic acid in water, a buffer, an aqueous solution containing salt, an aqueous polar solvent solution, or a mixture thereof; and
dialyzing the nucleic acid dissolved solution in an aqueous solution containing a multivalent metal cation having a valence of 2 or more to obtain a gel having a liquid crystalline structure, of which main conponent is nucleic acid.

14. The method according to claim 13, wherein the nucleic acid dissolved solution is prepared by dissolving a water-soluble DNA in a buffer, an aqueous solution containing salt, an aqueous polar solvent solution, or a mixture thereof; and the step of obtaining a gel having a liquid crystalline structure includes the steps of filling and sealing the nucleic acid dissolved solution in a dialysis-membrane tube, immersing the nucleic acid dissolved solution filled and sealed in the tube in an aqueous solution containing a multivalent metal cation having a valence of 2 or more, and then dialyzing the nucleic acid dissolved solution to form in the tube the cylindrical liquid crystalline structure which is concentric and radially oriented from the center when observation is conducted on a cross section perpendicular to the longitudinal direction of the tube, and taking out the gel from the tube and then rinsing the gal with water to obtain a solid cylindrical gel or a tubular gel having a hollowed cylindrical center portion.

15. The method according to claim 14, further comprising the steps of forming a gel having a cylindrical liquid crystalline structure in a tube and then taking out the tube from an aqueous solution containing a multivalent metal cation having a valence of 2 or more into the atmosphere followed by leaving it to stand; and immersing the tube in the aqueous solution and dialyzing the nucleic acid dissolved solution to form a tubular gel having a concentric multilayered structure in the tube when observation is conducted on a cross section perpendicular to a longitudinal direction of the tube.

16. The method according to claim 13, wherein the nucleic acid dissolved solution is prepared by dissolving a water-soluble DNA in a buffer, an aqueous solution containing salt, an aqueous polar solvent solution, or a mixture thereof; and the step of obtaining a gel having a liquid crystalline structure includes the steps of dropping the nucleic acid dissolved solution into an aqueous solution containing a multivalent metal cation having a valence of 2 or more using a syringe, a nozzle, a spray, or a micropipette to form a dialysis membrane on the entire circumference of a liquid droplet in the aqueous solution while dialyzing the nucleic acid dissolved solution to form a spherical gel, which is radially oriented from the center in the dialysis membrane when observation is conducted on a radial cross section.

17. The method according to claim 16, further comprising the steps of forming a dialysis membrane on the entire circumference of a liquid droplet in an aqueous solution containing a multivalent metal cation having a valence of 2 or more and then taking out a spherical gel into the atmosphere, on which the dialysis membrane is formed on the entire circumference, from the aqueous solution, followed by leaving it to stand; and immersing the spherical gel left to stand in the aqueous solution and dialyzing the nucleic acid dissolved solution to form a spherical gel having a concentric multilayered structure in the dialysis membrane when observation is conducted on a radial cross section thereof.

18. The method according to claim 13, wherein the nucleic acid dissolved solution is prepared by dissolving a water-soluble DNA in a buffer, an aqueous solution containing salt, an aqueous polar solvent solution, or a mixture thereof; and
the step of obtaining a gel having a liquid crystalline structure includes the steps of flattening the nucleic acid dissolved solution, which is dropped on a first plate and then covered with another second plate; and immersing the flattened nucleic acid dissolved solution sandwiched between the first plate and the second plate in an aqueous solution containing a multivalent metal cation having a valence of 2 or more to form a dialysis membrane on a portion of the flattened nucleic acid dissolved solution without being covered with first and second plates in the aqueous solution, while making the flattened nucleic acid dissolved solution into a plate or a film by dialying the nucleic acid dissolved solution with the dialysis membrane to form a plate-shaped or film-shaped gel in the dialysis membrane, which is concentric and radially oriented from the center when observation is conducted on a plate surface or a film surface; and taking out the gel from the first and second plates and rinsing the gel with water to obtain a plate-shaped or film-shaped gel.

19. The method according to claim 18, further comprising the steps of: forming a gel having a plate-shaped or film-shaped liquid crystalline structure between first and second plates in an aqueous solution containing a multivalent metal cation having a valence of 2 or more and then taking out the first and second plates from the aqueous solution containing the multivalent metal cation having the valence of 2 or more into the atmosphere followed by leaving them to stand; and immersing the first and second plates left to stand in the aqueous solution and dialyzing the nucleic acid dissolved solution to form a plate-shaped or film-shaped gel having a concentric multilayered structure between the first plate and second plate when observation is conducted on a plate surface or a film surface.

20. The method according to claim 13, wherein the nucleic acid dissolved solution is prepared by dissolving a water-soluble DNA in a buffer, an aqueous solution containing salt, an aqueous polar solvent solution, or a mixture thereof; and
the step of obtaining a gel having a liquid crystalline structure includes the steps of: extruding the nucleic acid dissolved solution from a syringe or nozzle into an aqueous solution containing a metal cation having a valence of 2 or more to form a dialysis membrane on the entire circumference of a rod-like or fibrous body in the aqueous solution while dialyzing the nucleic acid dissolved solution to form a gel having a rod-like or fibrous liquid crystalline structure, which is concentric and radially oriented from the center when observation is conducted on the cross section thereof perpendicular to the longitudinal direction, in the dialysis membrane; and taking out a gel from the aqueous solution and then rinsing the gel with water to obtain a solid or hollow rod-like or fibrous gel.

21. The method according to claim 20, further comprising the steps of:
forming a dialysis membrane on the entire circumference of a rod-like or fibrous body in an aqueous solution containing a multivalent metal cation having a valence of 2 or more and then taking out the rod-like or fibrous gel with the dialysis membrane formed on the entire circumference from the aqueous solution into the atmosphere followed by leaving to stand; and immersing the rod-like or fibrous gel and dialyzing the nucleic acid dissolved solution to form a rod-like or fibrous gel having a concentric multilayered structure in the dialysis membrane when observation is conducted on the cross section perpendicular to the longitudinal direction.
